# EUROPEAN PATENT APPLICATION

(11) **EP 1 510 208 A1**
(43) Date of publication of application: **02.03.2005**
(21) Application number: 03292077.9
(22) Date of filing: 22.08.2003
(51) Int. Cl.: A61K 31/155, A61K 31/366, A61K 31/404, A61K 31/40, A61K 31/506, A61P 3/06, A61P 3/10, A61P 9/00

(54) **Pharmaceutical composition comprising a combination of metformin and statin**

(71) Applicant: Fournier Laboratories Ireland Limited, Carrigtwohill, Co. Cork (IE)
(72) Inventor: Junien, Jean-Louis, 92310 Sevres (FR); Edgar, Alan, 21490 Saint Julien (FR)
(74) Representative: Nevant, Marc

(57) **Abstract**

A pharmaceutical composition comprising: (i) metformin, optionally in the form of one of its pharmaceutically acceptable salts, (ii) a statin, optionally in the form one of its pharmaceutically acceptable salts, and optionally one or more pharmaceutically acceptable excipients, is suitable for use in the treatment of hyperglycemia non-insulin-dependent diabetes, dyslipidemia, hyperlipidemia, hypercholesterolemia, and obesity.

## Description

This invention relates to therapeutically effective compositions and methods for treatment of patients with dyslipidemia, hyperlipidemia, hypercholesterolemia, hyperglycaemia, obesity and related conditions comprising a combination in one dosage form of metformin and of a hydroxymethylglutaryl coenzyme A (HMG-CoA) reductase inhibitor or statin, such as, for example, pravastatin, lovastatin, simvastatin, atorvastatin, cerivastatin or fluvastatin, formulated together to provide simultaneously a therapeutically effective amount of the hydroxymethylglutaryl coenzyme A reductase inhibitor and a therapeutically effective amount of the metformin taken into the blood of a patient in need of treatment. The compositions of this invention are useful to reduce the hyperglycaemia of non-insulin-dependent diabetes, dyslipidemia, hyperlipidemia, hypercholesterolemia, obesity.

In humans, cholesterol and triglycerides (TG) are part of lipoprotein complexes in the bloodstream, and can be separated via ultracentrifugation into high-density lipoprotein (HDL), intermediate-density lipoprotein (IDL), low-density lipoprotein, (LDL) and very-low-density lipoprotein (VLDL) fractions. Cholesterol and triglycerides are synthesized in the liver, incorporated into VLDL, and released into the plasma. High levels of total cholesterol (total-C), LDL-C, and apolipoprotein B (apo-B, a component of a membrane complex for LDL-C) promote human atherosclerosis, and decreased levels of HDL-C and its transport complex, apolipoprotein A, are associated with the development of atherosclerosis. Cardiovascular morbidity and mortality in humans can vary directly with the level of total-C and LDL-C and inversely with the level of HDL-C.

Orally administered statins are hydroxymethylglutaryl coenzyme A (HMG-CoA) reductase inhibitors that are used in patients to lower low density lipoprotein (LDL) cholesterol. Complimentary to this are orally administered fibrates which are used in patients to decrease lipoproteins rich in triglycerides, to increase high density lipoprotein (HDL), and to decrease atherogenic-dense LDL. Patients who take statins or fibrates are frequently on diets with low and variable fat content.

HMG-CoA reductase (3-hydroxy-3-methylglutaryl-coenzyme A) is the microsomal enzyme that catalyses the rate limiting reaction in cholesterol biosynthesis (Mevalonate). According to the present invention, a statin is an HMG-CoA reductase inhibitor that inhibits HMG-CoA reductase, and therefore inhibits or interferes with the synthesis of cholesterol. Inhibition of cholesterol synthesis can lead to a reduction in blood cholesterol levels.

A large number of naturally or synthetically obtained or synthetically modified compounds have been found to inhibit HMG-CoA reductase. These compounds form a category of agents useful for practicing the present invention. Traditionally these agents have been used to treat individuals with hypercholesterolemia.

Metformin is mainly known for its anti-hyperglycaemic activity and is widely used in the treatment of non-insulin-dependent diabetes. In the case of insulin-dependent diabetes, metformin is also administered to the patient in combination with insulin.

Surprisingly, the present inventors have discovered that the combination of metformin with a statin leads to a significant improvement of the hyperglycaemia in a diabetic patient suffering from non-insulin-dependent diabetes. More specifically, a synergistic effect has been obtained by combined administration of metformin and of a statin. The combination of metformin with a statin was never described in the prior art.

Thus, the invention relates to a pharmaceutical composition comprising, as active principles, (i) metformin, and (ii) a statin, in combination with one or more pharmaceutically acceptable excipients.

This composition is more particularly suitable for reducing the hyperglycaemia in non-insulin-dependent diabetes patients and to improve insulin resistance. It can also be used in non-dyslipidaemic patients or in dyslipidaemic patients.

The composition of the present invention includes statins, which are commercially available, such as lovastatin and mevinolin disclosed in U.S Pat. No. 4,231,938, pravastatin and pravastatin sodium disclosed in U.S. Pat. No. 4,346,227, fluvastatin and fluvastatin sodium and XU 62-320 disclosed in EP 0 114 027 and U.S. Pat. No. 4,739,073, atorvastatin disclosed in U.S. Pat. No. 5,273,995, itavastatin also known as NK-104 disclosed in EP304063, mevastatin disclosed in U.S. Pat. No. 3,983,140, rosuvastatin, velostatin and synvinolin and simvastatin disclosed in U.S. Pat. Nos. 4,448,784 and 4,450,171, cerivastatin, pitivastatin and numerous others described in U.S. Pat. Nos. 5,622,985, 5,135,935, 5,356,896, 4,920,109, 5,286,895, 5,262,435, 5,260,332, 5,317,031, 5,283,256, 5,256,689, 5,182,298, 5,369,125, 5,302,604, 5,166,171, 5,202,327, 5,276,021, 5,196,440, 5,091,386, 5,091,378, 4,904,646, 5,385,932, 5,250,435, 5,132,312, 5,130,306, 5,116,870, 5,112,857, 5,102,911, 5,098,931, 5,081,136, 5,025,000, 5,021,453, 5,017,716, 5,001,144, 5,001,128, 4,997,837, 4,996,234, 4,994,494, 4,992,429, 4,970,231, 4,968,693, 4,963,538, 4,957,940, 4,950,675, 4,946,864, 4,946,860, 4,940,800, 4,940,727, 4,939,143, 4,929,620, 4,923,861, 4,906,657, 4,906,624, RE36,520, and U.S. Pat. No. 4,897,402, the disclosures of which patents are incorporated herein by reference. The present invention is not limited to these statins.

Lovastatin, an inactive lactone, is a white, nonhygroscopic crystalline powder isolated from a strain of Aspergillus terreus that is insoluble in water and sparingly soluble in ethanol, methanol, and acetonitrile. Lovastatin is hydrolysed after oral ingestion to the corresponding (beta)-hydroxyacid. This metabolite is an inhibitor of 3-hydroxy-3-methylglutaryl-coenzyme A (HMG-CoA) reductase. When formulated for oral administration as Mevacor, tablets can contain 10 to 40 mg of lovastatin together with pharmaceutically acceptable excipients such as cellulose, lactose, magnesium stearate, starch, and butylated hydroxyanisole as a preservative. When taken separately, lovastatin can treat related hyperlipidemia such as reducing plasma total-C, LDL-C, total-C/HDL-C ratio and LDL-C/HDL-C ratio as well as increasing HDL-C, and modestly decreasing VLDL-C and plasma triglycerides TG. Mevacor can lower total-C and LDL-C to target levels, and reduce elevated total-C and LDL-C levels in patients with primary hypercholesterolemia (Types IIa and IIb). Single daily doses given in the evening can be more effective than the same dose given in the morning, perhaps because cholesterol is synthesized mainly at night. A recommended starting dose of Mevacor is preferably given with a meal. 20 mg once a day can be given with the evening meal. Storage between 5-30°C. (41-86°F.) is preferred.

Fluvastatin (also known as fluvastatin sodium), a synthetic HMG-CoA reductase inhibitor, is a white to pale yellow, hygroscopic powder soluble in water, ethanol and methanol. When formulated for oral administration as Lescol TM, capsules can contain 20 to 40 mg of fluvastatin together with pharmaceutically acceptable excipients such as gelatin, magnesium stearate, microcrystalline cellulose, pregelatinized starch, red iron oxide, sodium lauryl sulfate, talc, titanium dioxide, yellow iron oxide and other ingredients. Fluvastatin sodium reduces Total-C, LDL-C, and apolipoprotein B, and moderately reduces triglycerides (TG) while producing an increase in HDL-C of variable magnitude. Following oral administration, fluvastatin is absorbed rapidly and completely with peak concentrations reached in less than 1 hour. Administration with food reduces the rate but not the extent of absorption. Fluvastatin sodium is indicated as an adjunct to diet in the treatment of elevated total cholesterol (Total-C), LDL-C, TG and Apo B levels in patients with primary hypercholesterolemia and mixed dyslipidemia (Frederickson Type IIa and IIb). It is also indicated to slow the progression of coronary atherosclerosis in patients with coronary heart disease as part of a treatment strategy to lower total and LDL cholesterol to target levels.

Atorvastatin (or Atorvastatin calcium 2: 1) is a white to off-white crystalline trihydrate powder that is insoluble in aqueous solutions of pH 4 and below, and is very slightly soluble in distilled water, pH 7.4 phosphate buffer, and acetonitrile, slightly soluble in ethanol, and freely soluble in methanol. When formulated in Lipitor TM tablets for oral administration, tablets can contain 10 to 80 mg of atorvastatin as well as pharmaceutically acceptable excipients such as calcium carbonate, USP; candelilla wax, FCC; croscarmellose sodium, NF; hydroxypropyl cellulose, NF; lactose monohydrate, NF; magnesium stearate, NF; microcrystalline cellulose, NF; Opadry White YS-1-7040 (hydroxypropylmethylcellulose, polyethylene glycol, talc, titanium dioxide): polysorbate 80, NF; and simethicone emulsion. Atorvastatin can reduce total-C, LDL-C, and apo B in patients with homozygous and heterozygous familial hypercholesterolemia, nonfamilial forms of hypercholesterolemia, and mixed dyslipidemia. Atorvastatin can also reduce VLDL-C and TG and produces variable increases in HDL-C and apolipoprotein A-1. Atorvastatin can reduce total-C, LDL-C, VLDL-C, apo B, TG, and non-HDL-C, and can increase HDL-C in patients with isolated hypertriglyceridemia. Atorvastatin can reduce intermediate density lipoprotein cholesterol (IDL-C) in patients with dysbetalipoproteinemia. Food decreases the rate and extent of drug absorption as assessed by Cₘₐₓ and AUC, but LDL-C reduction is similar whether atorvastatin is given with or without food. Atorvastatin can be administered as a single dose at any time of the day, with or without food. Atorvastatin can reduce total-C, LDL-C, VLDL-C, apo B, and TG, and can increase HDL-C in patients with hypercholesterolemia and mixed dyslipidemia.

Simvastatin is a white to off-white, nonhygroscopic, crystalline powder that is practically insoluble in water, and freely soluble in chloroform, methanol and ethanol. Simvastatin is derived synthetically from a fermentation product of Aspergillus terreus. After oral ingestion, simvastatin, which is an inactive lactone, is hydrolysed to the corresponding (beta)-hydroxyacid form, which is an inhibitor of 3-hydroxy-3-methyl-glutaryl-coenzyme A (HMG-CoA) reductase. When formulated as Zocor TM for oral administration, tablets can contain 5 mg to 80 mg of simvastatin as well as pharmaceutically acceptable excipients such as cellulose, hydroxypropyl cellulose, hydroxypropyl methylcellulose, iron oxides, lactose, magnesium stearate, starch, talc, titanium dioxide as well as other ingredients including butylated hydroxyanisole which can be added as a preservative. Simvastatin shows no fed-fasted effect when administered immediately before a low-fat meal. Simvastatin can reduce total-C, LDL-C, total-C/HDL-C ratio, and LDL-C/HDL-C ratio as well as decrease TG and increase HDL-C.

Cerivastatin (or Cerivastatin sodium) is a white to off-white hygroscopic amorphous powder that is soluble in water, methanol, and ethanol, and very slightly soluble in acetone. Cerivastatin sodium is a synthetic, enantiomerically pure competitive inhibitor of the enzyme 3-hydroxy-3-methylglutaryl-coenzyme A (HMG-CoA) reductase that catalyzes the conversion of HMG-CoA to mevalonate in an early and rate-limiting step in the biosynthesis of cholesterol. The inhibition of cholesterol biosynthesis reduces the level of cholesterol in hepatic cells, which stimulates the synthesis of LDL receptors and increases the uptake of cellular LDL particles. This can lead to a reduction in plasma cholesterol concentration. When formulated as Baycol TM, cerivastatin sodium tablets can contain 0.2 to 0.8 mg of cerivastatin sodium for oral administration and can be taken with or without food. Other tablet ingredients can include pharmaceutically acceptable excipients such as mannitol, magnesium stearate, sodium hydroxide, crospovidone, povidone, iron oxide yellow, methylhydroxypropylcellulose, polyethylene glycol, and titanium dioxide. In patients with hypercholesterolemia, cerivastatin sodium can produce reduced levels of plasma total cholesterol, LDL-C, and apolipoprotein B, VLDL-C and plasma triglycerides and increases plasma HDL-C and apolipoprotein A-1. Cerivastatin systemic exposure (area under the curve, AUC) and Cₘₐₓ are not sensitive to a food effect, but once daily doses of 0.2 mg can be more efficacious than twice daily doses of 0.1 mg. Cerivastatin sodium can be effective as an adjunct to diet to reduce elevated Total-C, LDL-C, apo B, and TG and to increase HDL-C levels in patients with primary hypercholesterolemia and mixed dyslipidemia (Fredrickson Types IIa and IIb) when the response to dietary restriction of saturated fat and cholesterol and other non-pharmacological measures alone is inadequate.

Pravastatin (or pravastatin sodium) is a white to off-white, fine or crystalline powder. It is a relatively polar hydrophilic compound with a partition coefficient (octanol/water) of 0.59 at a pH of 7.0. It is soluble in methanol and water (>300 mg/mL), slightly soluble in isopropanol, and practically insoluble in acetone, acetonitrile, chloroform, and ether. When formulated as Pravachol TM for oral administration, tablets can contain 10 to 40 mg of pravastatin. Inactive ingredients can include pharmaceutically acceptable excipients such as croscarmellose sodium, lactose, magnesium oxide, magnesium stearate, microcrystalline cellulose, and povidone. A 10 mg tablet can also contain Red Ferric Oxide, a 20 mg tablet can also contain Yellow Ferric Oxide, and a 40 mg tablet can also contain Green Lake Blend (mixture of D&C Yellow No. 10-Aluminum Lake and FD&C Blue No. 1-Aluminum Lake).

Itavastatin is an inhibitor of HMG-CoA reductase and can be dosed in tablets containing from about 1 mg to about 20 mg, preferably from about 2 mg to about 10 mg.

Rosuvastatin is an inhibitor of HMG-CoA reductase and can be dosed in tablets containing from about 4 or 5 mg to about 10 or 20 mg, with reported doses of up to about 80 mg per day when formulated as Crestor TM.

Preferred statins in this invention are those useful for oral administration. According to the invention, the statin can be administered in the form of one of its pharmaceutically acceptable salts. Such salts, for example, can be formed between a positively charged substituent in a compound (e.g., amino) and an anion. Suitable anions include, but are not limited to, chloride, bromide, iodide, sulfate, nitrate, phosphate, citrate, methanesulfonate, trifluoroacetate, and acetate. Likewise, a negatively charged substituent in a compound (e.g., carboxylate) can form a salt with a cation. Suitable cations include, but are not limited to, sodium ion, potassium ion, magnesium ion, calcium ion, and an ammonium cation such as tetramethylammonium ion. Among the salts, the sodium, calcium are more particularly preferred.

If in the composition of the present invention, lovastatin is used, the amount is between 10 to 40 mg, and more preferably 20 mg per day. If fluvastatin is used, the amount is between 20 to 40 mg per day. If atorvastatin is used, the amount is between 10 to 80 mg, and more preferably 10 to 40 mg per day. If simvastatin is used, the amount is between 5 to 50 mg, more preferably 5 to 20 mg per day. If cerivastatin is used, the amount is between 0.1 to 0.8 mg, more preferably 0.1 to 0.3 mg per day. If pravastatin is used, the amount is between 10 to 40 mg, more preferably 20 mg per day. If itavastatin is used, the amount is between 1 to 20 mg, more preferably from 2 to 20 mg per day. If rosuvastatin is used, the amount is between 4 to 80 mg, more preferably 10 to 20 mg per day.

The amount of statin used in the composition of the invention is the amount mentioned before with respect to each specific statin. This amount can be chosen between 0.1 mg to 100 mg depending on the specific statin used.

According to the invention, the metformin can be administered in the form of one of its pharmaceutically acceptable salts, such as the hydrochloride, acetate, benzoate, citrate, fumarate, embonate, chlorophenoxyacetate, glycolate, palmoate, aspartate, methanesulphonate, maleate, parachlorophenoxyisobutyrate, formate, lactate, succinate, sulphate, tartrate, cyclohexanecarboxylate, hexanoate, octonoate, decanoate, hexadecanoate, octodecanoate, benzenesulphonate, trimethoxybenzoate, paratoluenesulphonate, adamantanecarboxylate, glycoxylate, glutamate, pyrrolidonecarboxylate, naphthalenesulphonate, 1-glucosephosphate, nitrate, sulphite, dithionate or phosphate.

Among these salts, the hydrochloride, fumarate, embonate and chlorophenoxyacetate are more particularly preferred.

The pharmaceutically acceptable salts of metformin are obtained in a manner, which is known per se, by the action of metformin on the corresponding acid.

A once a day amount of metformin is generally from 200 mg to 2000 mg. The most common composition contains from 500 mg and 850 mg of metformin and are to be taken twice or three times a day.

Thus, if the compositions of the invention contain 500 or 850 mg and are to be taken more than once a day, then the amount of statin will be adjusted consequently.

The amount of metformin and the amount of statin together provide a dosage or amount of the combination that is sufficient to constitute an effective amount of the combination. The effective amount can be a glycaemic or lipidaemic disorder or disease suppressing treatment or prevention effective amount.

As used herein, an "effective amount" means the dose or effective amount to be administered to a patient. The dose or effective amount to be administered to a patient and the frequency of administration to the subject can be readily determined by one of ordinary skill in the art by the use of known techniques and by observing results obtained under analogous circumstances. In determining the effective amount or dose, a number of factors are considered by the attending diagnostician, including but not limited to, the potency and duration of action of the compounds used; the nature and severity of the illness to be treated as well as on the sex, age weight, general health and individual responsiveness of the patient to be treated, and other relevant circumstances.

The compositions of the invention contain therapeutically effective amounts of the various active principles. The ratios of the respective amounts of statin, or one of its pharmaceutically acceptable salts, and of metformin, or one of its pharmaceutically acceptable salts, thus vary in consequence. These ratio of statin, or its pharmaceutically acceptable salt, to metformin, or its pharmaceutically acceptable salt, can vary from 1:2 to 1:20000. It is preferred that the weight ratio of the amount of statin to the amount of metformin that is administered to the subject is within a range of from about 1:2, preferably 1:4 to 1:2000 and more preferably 1:5 to 1:2000. The ratio of statin to metformin in the composition of the invention will vary if the present pharmaceutical composition is to be taken more than once a day, or if it is to be only taken once a day.

The expression "therapeutically-effective" indicates the capability of an agent to prevent, or improve the severity of, the disorder, while avoiding adverse side effects typically associated with alternative therapies. The expression "therapeutically-effective" is to be understood to be equivalent to the expression "effective for the treatment, prevention, or inhibition", and both are intended to qualify the amount of each agent for use in the combination therapy, which will achieve the goal of improvement in the severity of non insulin dependent diabetes.

The metformin amount is that sufficient to constitute an effective amount of the combination. Preferably, such amount would be sufficient to provide a therapeutically effective amount of the combination. The therapeutically effective amount can also be described herein as a hyperglycaemic treatment or prevention effective amount of the combination, or as a hypercholesterolemia or dyslipidemia or disease suppressing treatment or prevention effective amount.

The combination of metformin and a statin can be supplied in the form of a novel therapeutic composition that is believed to be within the scope of the present invention. The relative amounts of each component in the therapeutic composition may be varied and may be as described just above. The metformin and statin that are described above can be provided in the therapeutic composition so that the preferred amounts of each of the components are supplied by a single dosage, a single injection or a single capsule for example, or, by up to two, or more, single dosage forms.

When the novel combination is supplied along with a pharmaceutically acceptable carrier, a pharmaceutical composition is formed. A pharmaceutical composition of the present invention is directed to a composition suitable for the prevention or treatment of hyperglycaemia, non insulin dependent diabetes, hyperlipidemia, dyslipidemia, hypercholesterolemia or obesity. The pharmaceutical composition comprises a pharmaceutically acceptable carrier, a statin and a metformin.

Pharmaceutically acceptable carriers include, but are not limited to, physiological saline, Ringer's, phosphate solution or buffer, buffered saline, and other carriers known in the art. Pharmaceutical compositions may also include stabilizers, anti-oxidants, colorants, and diluents, or any pharmaceutical excipients.

The method and combination of the present invention are useful for, but not limited to, the prevention, inhibition and treatment of hyperglycaemia, non insulin dependent diabetes, dyslipidaemia, hyperlipidemia, hypercholesterolemia, obesity and cardiovascular diseases.

As described above, an embodiment of the present invention comprises a pharmaceutical composition, comprising a therapeutically-effective amount of a combination of metformin and a statin in association with at least one pharmaceutically-acceptable carrier, adjuvant or diluent and, if desired, other active ingredients.

The expressions "combination therapy" and "administration with" in defining the use of a metformin and a statin is intended as to embrace co-administration of these agents in a substantially simultaneous manner, such as in a single capsule or dosage device having a fixed ratio of these active agents or in multiple, separate capsules or dosage devices that can be taken together contemporaneously.

The compositions of the invention are preferably administered enterally or parenterally (parenteral administration includes subcutaneous, intramuscular, intradermal, intramammary, intravenous, and other administrative methods known in the art), or better still orally, although the other routes of administration, for instance such as rectal administration, are not excluded.

For preparing oral pharmaceutical compositions from the compounds of this invention, inert, pharmaceutically acceptable carriers can be either solid or liquid. Solid form preparations include powders, tablets, coated tablets, dragees, troches, lozenges, dispersible granules, capsules, and sachets. Compositions for oral use may be prepared according to any method known in the art of manufacture of pharmaceutical compositions.

A solid carrier can be one or more substances which may also act as diluents, flavouring agents, solubilizers, lubricants, suspending agents, binders, or tablet disintegrating agents; it can also be an encapsulating material.

In powders, the carrier is a finely divided solid, which is in a mixture with the finely divided active component. In tablets, the active compound is mixed with the carrier having the necessary binding properties in suitable proportions and compacted in the shape and size desired.

Suitable carriers may be, for example, inert diluents, such as magnesium carbonate, calcium stearate, magnesium stearate, talc, lactose, sugar, pectin, dextrin, starch, tragacanth, methyl cellulose, sodium carboxymethyl cellulose, and the like.

The present invention also includes the formulation of metformin and statin with encapsulating material as a carrier providing a capsule in which metformin and statin (with or without other carriers) is surrounded by a carrier, which is thus in association with metformin and statin. In a similar manner, sachets are also included. Tablets, powders, sachets, and capsules can be used as solid dosage forms suitable for oral administration.

The tablets may be uncoated or coated by known techniques to delay disintegration and adsorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredients are mixed with inert solid diluent, for example, calcium carbonate, calcium phosphate or kaolin, or as soft gelatin capsules wherein the active ingredients are present as such, or mixed with water or an oil medium, for example, arachid oil, liquid paraffin, or olive oil.

Aqueous suspensions can be produced that contain the active compounds in admixture with excipients suitable for the manufacture of aqueous suspensions. Such excipients are suspending agents, for example, sodium carboxymethylcellulose, methylcellulose, hydroxypropylmethylcellulose, sodium alginate, polyvinylpyrrolidone gum tragacanth and gum acacia; dispersing or wetting agents may be naturally-occuring phosphatides, for example lecithin, or condensation products of an alkylene oxide with fatty acids, for example polyoxyethylene stearate, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyoxyethylene sorbitan monooleate.

The aqueous suspensions may also contain one or more preservatives, for example, ethyl or n-propyl p-hydroxybenzoate, one or more coloring agents, one or more flavoring agents, one or more sweetening agents.

Oily suspensions may be formulated by suspending the active compounds in an omega-3 fatty acid, a vegetable oil, for example arachid oil, olive oil, sesame oil or coconut oil, or in a mineral oil such as liquid paraffin. The oily suspensions may contain a thickening agent, for example, beeswax, hard paraffin or cetyl alcohol.

Sweetening agents and flavoring agents may be added to provide a palatable oral preparation. These compositions may be added to provide a palatable oral preparation. These compositions may be preserved by the addition of an antioxydant such as ascorbic acid.

Dispersible powders and granules suitable for preparation of an aqueous suspension by the addition of water provide the active compounds in admixture with a dispersing or wetting agent, a suspending agent and one or more preservatives. Suitable dispersing or wetting agents and suspending agents are exemplified by those already mentioned above. Additional excipients, for example sweetening, flavoring and coloring agents, may also be present.

Syrups and elixirs containing the novel combination may be formulated with sweetening agents. Such formulations may also contain a demulcent, a preservative and flavoring and coloring agents.

Formulations developed for metformin can be used for the pharmaceutical composition of the invention containing metformin and a statin. Such formulation of the metformin are described in the following patents: Gastric retentive (WO 9907342), controlled release metformin composition (WO 0236100), controlled release with unitary core (WO 9947125), treatment with 400 mg or below of metformin (US 6,100,300), novel salts of metformin (WO 9929314), biphasic controlled release delivery system (WO 9947128), metformin preparation (WO 9608243), gastric retentive (WO 9855107), controlled release (WO 0103964 and WO 0239984), metformin tablet (WO 03004009), sustained release composition (WO 02067905), controlled release composition (WO 0211701), gastroretentive (WO 0006129), solid carriers for improved delivery (WO 0137808), coating for sustained release composition (WO 02085335), modified release composition (WO 03002151), liquid formulation of metformin (WO 0247607), controlled release device (WO 02094227), metformin quick release tablet (JP 2002326927).

Among those formulations, the metformin once a day formulation are preferred.

Liquid form preparations include solutions, suspensions and emulsions suitable for oral administration. Aqueous solutions for oral administration can be prepared by dissolving the active compounds in water and adding suitable flavorants, colouring agents, stabilizers, and thickening agents as desired. Ethanol, propylene glycol and other pharmaceutically acceptable non-aqueous solvents may be added to improve solubility of the active compounds. Aqueous suspensions for oral use can be made by dispersing the finely divided active compounds in water together with a viscous material such as natural or synthetic gums, resins, methyl cellulose, sodium carboxymethyl cellulose, and other suspending agents known to the pharmaceutical formulation art.

Preferably, the pharmaceutical preparation is in unit dosage form. In such form, the preparation is divided into unit doses containing appropriate quantities of the active compounds. The unit dosage form can be a packaged preparation, the package containing discrete quantities of the preparation, for example, packeted tablets, capsules, and powders in vials or ampoules. The unit dosage form can also be a capsule, cachet, or tablet itself, or it can be the appropriate number of any of these packaged forms.

The subject combinations can also be administered parenterally either subcutaneously, or intravenously, or intramuscularly, or intrasternally, or by infusion techniques, in the form of sterile injectable aqueous or olagenous suspensions. Such suspensions may be formulated according to the known art using those suitable dispersing of wetting agents and suspending agents which have been mentioned above, or other acceptable agents. The sterile injectable preparation may also be a sterile injectable solution or suspension in a non-toxic parenterally-acceptable diluent or solvent, for example as a solution in 1,3-butanediol. Among the acceptable vehicles and solvent that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. In addition, n-3 polyunsaturated fatty acids may find use in the preparation of injectables.

The subject combination can also be administered by inhalation, in the form of aerosols or solutions for nebulizers, or rectally in the form of suppositories prepared by mixing the drug with a suitable non irritating excipient, which is solid at ordinary temperature but liquid at the rectal temperature and will therefore melt in the rectum to release the drug. Such materials are cocoa butter and polyethylene glycols.

Preferably, the subject composition is a controlled release composition.

Daily dosages can vary within wide limits and will be adjusted to the individual requirements in each particular case. In general, for administration to adults, an appropriate daily dosage has been described above, although the limits that were identified as being preferred may be exceeded if expedient. The daily dosage can be administered as a single dosage or divided dosages.

The present invention further comprises kits that are suitable for use in performing the methods of treatment described above. In one embodiment, the kit contains a first dosage form comprising metformin in one or more of the forms identified above and a second dosage form comprising one or more of the statin identified above, for a simultaneous administration, in quantities sufficient to carry out the methods of the present invention.

The following example describes an embodiment of the invention. Other embodiments within the scope of the claims herein will be apparent to one skilled in the art from consideration of the specification or practice of the invention as disclosed herein. It is intended that the specification, together with the example, be considered to be exemplary only, with the scope and spirit of the invention being indicated by the claims, which follow the example.

### Example

The synergism of action was proven using an animal model. Zucker obese (fa/fa) rats were used to have a mimic of non-insulin-dependent diabetes (NIDD). The action of lovastatin alone, of metformin alone and of the combination lovastatin+metformin was evaluated in terms of triglycerides, total cholesterol, High Density Lipoprotein-C (HDL C), glucose and insulin. The rats were given the treatment for five consecutive days. Blood samples were collected three days before and five days after the beginning of the treatments in order to measure triglycerides, total cholesterol, HDL C, glucose and insulin levels.

The procedure followed was:

Four groups of 8 rats were formed:
- a vehicle group,
- a group who received a dose of 1mg/kg/day of lovastatin per os,
- a group who received a dose of 50 mg/kg twice a day (bid) of metformin per os,
- a group who received a dose of 1 mg/kg/day of lovastatin + 50mg/kg twice a day (bid) of metformin per os.

Statistical analysis consist in a one-way analysis of variance followed by multiple comparisons versus the vehicle group (Dunnett's test) to evaluate the significance results obtained, values are expressed as mean ± S.E.M. A difference will be considered significant for p<0.05. Results are expressed in millimol per litter (mM) or nanomol per litter (nM).

The results are reported in table 1 below:

**Table 1.**

| **Effects of lovastatin alone, metformin alone and lovastatin+metformin on serum biomarkers in Zucker obese (fa/fa) rats dosed per os for five days.** | | | | | | | | | | |
|---|---|---|---|---|---|---|---|---|---|---|
| Treatment mg/kg | Triglycerides (mM) | | Total Cholesterol (mM) | | HDL (mM) | C | Glucose (mM) | | Insulin (nM) | |
| | T0 | T5 | T0 | T5 | T0 | T5 | T0 | T5 | T0 | T5 |
| Vehicle | 4.22 | 4.79 | 2.42 | 2.53 | 1.95 | 2.12 | 8.44 | **8.28** | 0.67 | 0.49 |
| | *0.35* | *0.32* | *0.06* | *0.06* | *0.04* | *0.06* | *0.62* | *0.36* | *0.15* | *0.05* |
| | | | | | | | | | | |
| Lovastatin | 4.18 | 4.79 | 2.38 | 2.54 | 2.00 | 2.16 | 7.88 | **8.21** | 0.35 | 0.39 |
| 1 mg/kg, p.o. | *0.41* | *0.48* | *0.11* | *0.10* | *0.08* | *0.08* | *0.44* | *0.26* | *0.09* | *0.07* |
| | | | | | | | | | | |
| Metformin | 4.08 | 5.17 | 2.55 | 2.72 | 2.08 | 2.25 | 8.27 | **7.73** | 0.34 | 0.38 |
| 50 mg/kg, p.o. | *0.32* | *0.42* | *0.11* | *0.11* | *0.08* | *0.08* | *0.69* | *0.31* | *0.03* | *0.02* |
| | | | | | | | | | | |
| Lovastatin+Metformin | 4.10 | 4.40 | 2.40 | 2.43 | 1.96 | 2.07 | 7.69 | **7.00*** | 0.29 | 0.36 |
| 1+50 mg/kg, p.o. | *0.33* | *0.30* | *0.10* | *0.06* | *0.08* | *0.06* | *0.54* | *0.23* | *0.05* | *0.05* |

The results obtained show the synergism of action of lovastatin and metformin on glycemia.

Metformin used alone leads to a glycemia of 7.73 mM and lovastatin alone leads to a glycemia of 8.21 mM. The combination of metformin and lovastatin leads to a glycemia of 7.00 mM.

## Claims

1. A pharmaceutical composition comprising (i) metformin, (ii) a statin, and one or more pharmaceutically acceptable excipients.

2. A pharmaceutical composition according to claim 1, wherein the statin is chosen among lovastatin, fluvastatin, atorvastatin, simvastatin, pravastatin, itavastatin and rosuvastatin.

3. A pharmaceutical composition according to claim 1 or 2, wherein metformin is in the form of a salt chosen among the hydrochloride, acetate, benzoate, citrate, fumarate, embonate, chlorophenoxyacetate, glycolate, palmoate, aspartate, methanesulphonate, maleate, parachlorophenoxyisobutyrate, formate, lactate, succinate, sulphate, tartrate, cyclohexanecarboxylate, hexanoate, octonoate, decanoate, hexadecanoate, octodecanoate, benzenesulphonate, trimethoxybenzoate, paratoluenesulphonate, adamantanecarboxylate, glycoxylate, glutamate, pyrrolidonecarboxylate, naphthalenesulphonate, 1-glucosephosphate, nitrate, sulphite, dithionate and phosphate.

4. A pharmaceutical composition according to any of claims 1 to 3, wherein metformin is in the form of a salt which is chosen among the hydrochloride, fumarate, embonate, and chlorophenoxyacetate.

5. A pharmaceutical composition according to any of claims 1 to 4, wherein the statin is in the form of a salt chosen among the chloride, bromide, iodide, sulfate, nitrate, phosphate, citrate, methanesulfonate, trifluoroacetate, acetate, sodium ion, potassium ion, magnesium ion, calcium ion, and an ammonium cation such as tetramethylammonium ion.

6. A pharmaceutical composition according to any of claims 1 to 5, wherein said composition contains from 0.1 to 100 mg of a statin.

7. A pharmaceutical composition according to any of claims 1 to 6, wherein said composition contains from 200 to 2000 mg of metformin.

8. A pharmaceutical composition according to any of claims 1 to 7, wherein the weight ratio of statin to metformin is 1:2 to 1:20000.

9. A pharmaceutical composition according to any of claims 1 to 8, wherein said composition is in the form of powders, tablets, coated tablets, dragees, troches, lozenges, dispersible granules, capsules, and sachets.

10. A pharmaceutical composition according to any of claims 1 to 8, wherein said composition is in the form of solutions, suspensions and emulsions.

11. A pharmaceutical composition according to any of claims 1 to 10, wherein the pharmaceutical composition is a controlled release composition.

12. A composition comprising metformin and statin as a pharmaceutical composition.

13. The use of metformin and a statin for the manufacturing of a pharmaceutical composition for prevention, inhibition or treatment of hyperglycaemia, non insulin dependent diabetes, dyslipidaemia, hyperlipidemia, hypercholesterolemia, obesity and cardiovascular diseases.

14. The use according to claim 13, wherein the pharmaceutical composition is administered orally.

15. The use of metformin and a statin for the manufacturing of a kit comprising metformin, or one of its pharmaceutically acceptable salts, and a statin, or one of its pharmaceutically acceptable salts, for the simultaneous co-administration of metformin and a statin.
